(19) Europäisches Patentamt
European Patent Office
Office européen des brevets

(11) Publication number: 0 253 912 B1

(12) # EUROPEAN PATENT SPECIFICATION

(45) Date of publication of patent specification: 06.02.91 Bulletin 91/06

(21) Application number: 86110064.2

(22) Date of filing: 22.07.86

(51) Int. Cl.$^5$: **C07H 3/04**, C07H 3/06, C07H 15/04, A61K 31/70, C12P 19/12, C12P 19/00, G01N 33/569, C07H 15/10, // C12N15/00

(54) **Oligosaccharides, method for their manufacture and their use.**

(43) Date of publication of application: 27.01.88 Bulletin 88/04

(45) Publication of the grant of the patent: 06.02.91 Bulletin 91/06

(84) Designated Contracting States: AT BE CH DE FR GB IT LI LU NL SE

(56) References cited:
CARBOHYDRATE RESEARCH, vol. 134, 1984, pages 157-166, Elsevier, Science Publishers B.V., Amsterdam, NL; H. BRADE et al.: "Spectroscopic analysis of a 3-deoxy-D-manno-2-octulosonic acid (KDO)-disaccharide from the lipopolysaccharide of a Salmonella godesberg Re mutant"

(56) References cited:
SCIENCE, vol. 220, no. 4603, 17th June 1983, pages 1279-1281; M. NURMINEN et al.: "The genus-specific antigen of chlamydia: Resemblance to the lipopolysaccharide of enteric bacteria"

(73) Proprietor: Brade, Helmut, Dr. med.
Seekoppel 16
D-2361 Kükels (DE)

(72) Inventor: Brade, Helmut, Dr. med.
Seekoppel 16
D-2361 Kükels (DE)

(74) Representative: TER MEER - MÜLLER - STEINMEISTER & PARTNER
Mauerkircherstrasse 45
D-8000 München 80 (DE)

EP 0 253 912 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Field of the Invention

This invention relates to oligosaccharides, a method for their manufacture and their use as immunogens and antigens in the diagnosis and therapy of human and animal chlamydial infections.

### Background of the invention

Chlamydiae are pathogenic, obligatory intracellular parasites causing a variety of diseases in animals and humans.

There are two species, chlamydia trachomatis and Chlamydia psittaci Chlamydia trachomatis is a specific human pathogen, whereas Chlamydia psittaci is predominantly a pathogen of a variety of non-primate species. Diseases caused by Chlamydia trachomatis, present a major health problem throughout the world. In developing countries, Chlamydia trachomatis causes hyperendemic trachoma which is the world's leading cause of preventable blindness. In industralized nations, Chlamydia trachomatis is a sexually transmitted pathogen that causes an array of genital tract and associated infections whose incidence is increasing at epidemic rates. Chlamydiallike organisms have also been indentified in tissues of spiders (Coelotus luctuosus) and clams (Mercenarie mercenaria), suggesting that chlamydiae are ubiquitous parasites present throughout the animal kingdom. Samples of chlamydiae, such as those referred to above are commercially available from depositories, such as The American Type Culture Collection.

Chlamydiae have a unique developmental cycle including metabolically active, non-infectious, multiplying reticulate bodies and metabolically inactive but infectious elementary bodies. Surface components of these unique bacteria have been assumed to participate in the early steps during infection (adhesion and penetration) and may be responsible for the inhibition of phagosome-lysosome fusion, a characteristic feature of the chlamydial infection.

These surface components of Chlamydiae also represent antigens among which genus-, species-, and subspecies-specific antigens have been found, giving rise to the induction of specific antibodies during infection. Although there is a considerable knowledge on the serological and biological properties of these antigens little is known on the chemistry of these structures. One of the major antigens is a heatstabile, genus-specific glycolipid antigen which has been proposed to be similar to the lipopolysaccharide (LPS) of gram-negative bacteria. Recently, it has been shown definitely, by chemical means, that the glycolipids of Chlamydia trachomatis serotype L2 (Nurminen, M., E. Th. Rietschel, and H. Brade. 1985. Infect. Immun. 48 : 573-575) and of Chlamydia Psittaci strain PK 5082 (Brade L., S. Schramek, U. Schade and H. Brade. Infect. Immun., submitted.) are in fact typical lipopotysaccharides since they contain characteristic structural elements such as D-glucosamine, 3-deoxy-D-manno-2-octulosonic acid (KDO), and 3-hydroxy long chain fatty acids, components which have been detected in all lipopolysaccharides investigated so far. Further evidence for the similarity between chlamydial and enterobacterial lipopolysaccharide was obtained from serological investigations showing a crossreaction of chlamydial with enterobacterial lipopolysaccharide of the Re-chemotype and with lipopolysaccharide from Acinetobacter calcoaceticus.

Recently, Caldwell and Hitchcock have reported on a monoclonal antibody which recognizes a genus-specific epitope on the chlamydial lipopolysaccharide which does not react with Re lipopolysaccharide in western-blot analysis (Caldwell, H. D., and P. J. Hitchcock. 1984. Infect. Immun. 44 : 306-314). Thus, the chlamydial lipopolysaccharide expresses at least two antigenic determinants one of which is chlamydia-specific the other being similar to the Re antigenic determinant. These results have been confirmed by using polyclonal antisera against chlamydial and Re-type lipopolysaccharide in a passive hemolysis assay, and, moreover, the presence of two antibody specificities in polyclonal antisera against Chlamydiae has been demonstrated by absorption experiments. One of these antibodies reacted with both, chlamydial and Re lipopolysaccharide, the other was chlamydiaspecific. In addition, it has been shown that chlamydial lipopolysaccharide contains the lipid A antigenic determinant which, like in other bacteria, is cryptic in lipopolysaccharide and exposed only after acid hydrolysis (Brade, L., M. Nurminen, P. H. Mäkelä, and H. Brade. 1985. Infect. Immun. 48 : 569-572).

The lack of profound biochemical knowledge on surface structures of Chlamydiae is due to the fact that they cannot be propagated easily in large enough quantities to allow a structural analysis. Recently, Nano and Caldwell (Nano, F. E., and H. D. Caldwell. 1985. Science 228 : 742-744) reported the molecular cloning of a DNA fragment of Chlamydia trachomatis using the pUC8 plasmid as a vector. Transformants which contained the recombinant plasmid expressed, in addition to the parental lipopolysaccharide, a second, modified lipopolysaccharide population which was i) similarly sized as lipopolysaccharide of enterobacterial

Re mutants and, ii) expressed the aforementioned chlamydia-specific lipopolysaccharide epitope as analyzed by SDS-PAGE followed by silver staining or western-blot analysis. The authors hypothesized that the cloned DNA fragment encoded for a glycosyl transferase which added to the biosynthetically growing lipopolysaccharide a sugar which i) determines the chlamydia-specific epitope and ii) hinders the normal synthesis of the parent lipopolysaccharide.

These recombinants combine the ease of growth with the expression of the chlamydia-specific epitope and, are thus, a convenient source for the preparation of larger quantities of lipopolysaccharide, which allowed a structural investigation with regard to the genus-specific epitope of Chlamydiae.

## Summary of the invention

The inventors have investigated the chemical structure of chlamydial lipopolysaccharide with the intention of providing specific oligosaccharides that harbor the genus-specific epitope of chlamydial lipopolysaccharide and which can be used as specific inhibitors of antigen-antibody reactions.

During this investigation it has been found that by the hydrolysis of chlamydial lipopolysaccharide two oligosaccharides, i.e. the disaccharide disodium 8-O-(3-deoxyD-manno-2-octulopyranosyl)onate-3-deoxy-D-manno-2-octulosonate and the trisaccharide trisodium 4-O-{[8-O-(3-deoxy-D-manno-2-octulopyranosyl-)onate]-(3-deoxy-α-D-manno-2-octulopyranosyl) onate}-3-deoxy-D-manno-2-octulosonate, can be obtained, which comprise the genus-specific epitope of chlamydial lipopolysaccharide and are haptenic oligosaccharides which can be used as specific inhibitors of antigen-antibody reactions, as immunogens and as antigens in the diagnosis and therapy of human and animal chlamydial infections.

Subject matter of the present invention therefore are oligosaccharides having the general formula (I)

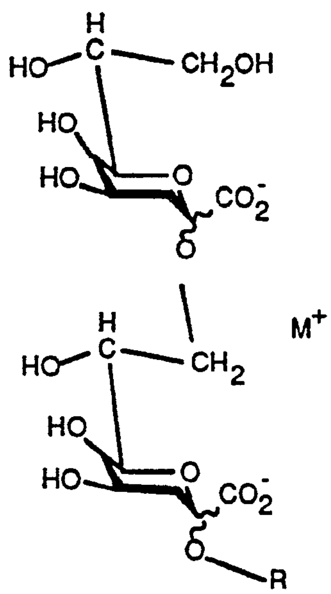

(I)

wherein R is $R^1$ or a group having the formula (II)

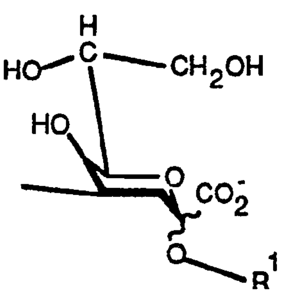

(II)

wherein $R^1$ is hydrogen or straight or branched $C_{1-18}$-alkyl, which may comprise a terminal hydroxyl, amino, carboxyl, carbonyl or allyl group, and M+ is a neutralizing cation, the glyco-protein conjugates thereof obtained with albumin, gamma-globulin or diphteria or tetanus toxoids and the polymer and copolymer derivatives of the compounds wherein $R^1$ comprises an allyl group.

The invention furtheron comprises a method for the manufacture of the oligosaccharides of the general formula (I), by chemical synthesis or by hydrolyzing a uniform salt of chlamydial lipopolysaccharide or lipopolysaccharides from recombinants expressing the genus-specific epitop of chlamydial LPS in an acetate buffer, dialyzing the hydrolysate against water, neutralising the dialysate, separating the oligosaccharides by liquid chromatography (gel-permeation, ion-exchange) or high-voltage paper electrophoresis (following the purity by gas-liquid chromatography and mass spectrometry) and if desired introducing the substituent $R^1$ into the disaccharide and trisaccharide obtained respectively by a method known per se and/or polymerizing or copolymerizing the derivatives of the compounds wherein $R^1$ comprises an allyl group.

The above oligosaccharides of the general formula (I) may be used as immunogens and antigens in the diagosis and therapy of human and animal chlamydial infections.

**Detailed description of the preferred embodiments**

The most preferred embodiments of the oligosaccharides of the above general formula (I) comprise the disaccharide of the following formula (III)

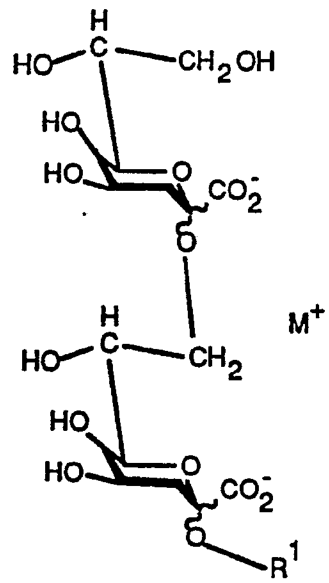

(III)

and the trisaccharide of the following formula (IV)

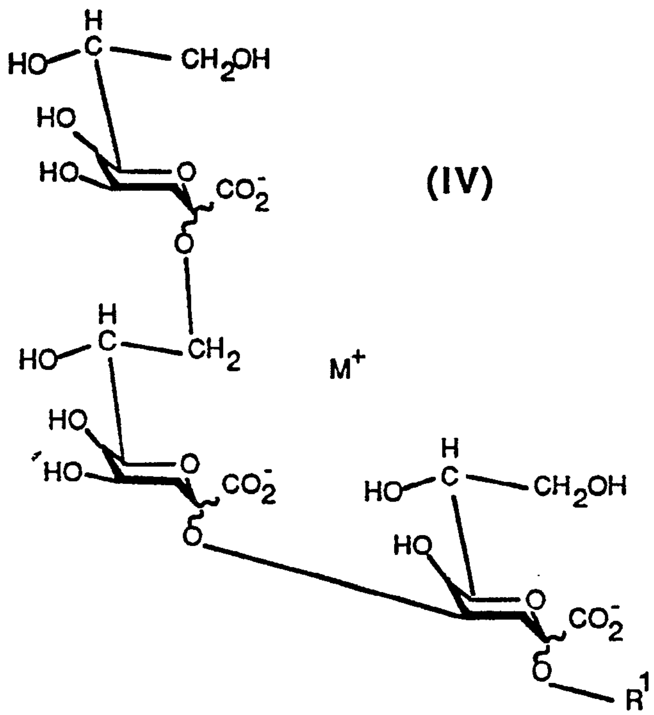

(IV)

wherein $R^1$ have the meanings above defined. $R^1$ therefore is hydrogen or a straight or branched $C_{1-18}$-alkyl, which may comprise a terminal hydroxyl, amino, carboxyl, carbonyl or allyl group, such as $C_{1-18}$-hydroxyalkyl, $C_{1-18}$-aminoalkyl, $C_{1-18}$-carboxyalkyl, aldehydoalkyl or $C_{2-18}$-alkenyl. More preferrably $R^1$ is hydrogen or a straight or branched $C_1$-$C_4$-alkyl, which may comprise a terminal hydroxyl, amino, carboxyl, carbonyl or allyl group. More preferrably $R^1$ is hydrogen methyl or allyl.

The neutralizing cation M+ can be a sodium, potassium, calcium or ammonium cation, which may comprise substituted ammonium cations as well, such as a trialkylammonium cation, such as a trimethylammonium cation.

Although the structure of the oligosaccharides of the present invention has been clarified apart from the anomeric configuration of the terminal 3-deoxy- D-manno-2-octulopyranosyl residue, the anomeric configurations are drawn by wave lines representing both anomers.

The oligosaccharides of the present invention can be manufactured either by chemical synthesis using methods known per se, such as those described by Unger (Anderson, L. and F. M. Unger. 1983. ACS Symp. Ser. 231 : 171-191 ; P. Kosma, J. Gass, T. Korosec, G. Schulz and F.M. Unger, 1986, J. Immunol. Immunopharmacol. VI-3/S :160).

A further method for the manufacture of the oligosaccharides of the present invention comprises the method according to claims 1 to 4, which is characterized by hydrolyzing a uniform salt of chlamydial lipopolysaccharide or lipopolysaccharides from recombinants expressing the genus-specific epitop of chlamydial LPS in an acetate buffer, dialyzing the hydrolysate against water, neutralising the dialysate, separating the oligosaccharides by liquid chromatography (gel-permeation, ionexchange) or high-voltage paper electrophoresis following the purity by gas-liquid chromatography and if desired introducing the substituent $R^1$ into the disaccharide and trisaccharide obtained respectively by a method known per se and/or polymerizing or copolymerizing the derivatives of the compounds wherein $R^1$ comprises an allyl group.

The uniform salt of lipopolysaccharide can be obtained by growing Chlamydia trachomatis or Chlamydia psittaci in tissue cultures or embryonated eggs or by growing recombinant bacteria which express in their lipopolysaccharide the genusspecific epitope of chlamydial lipopolysaccharide in a fermenter, extracting lipopolysaccharide from the purified elementary bodies or recombinant bacteria, respectively by the phenol-chloroform-petroleum ether method (Brade H., C. Galanos Eur. J. Biochem. 122, (1982) 233-237), purifying the extracted lipopolysaccharide by repeated ultracentrifugation and converting the purified lipopolysaccharide to the uniform triethylammonium salt after electrodialysis.

Chlamydia trachomatis and Chlamydia psittaci are well known microorganisms found in nature and which can be obtained from recognized depositories.

Recombinant bacteria, suitable to obtain Chlamydia-specified LPS can be prepared by the method described (Nano F.E. and H. D. Caldwell. 1985. Science 228 : 742-744).

The oligosaccharides of the present invention, wherein $R^1$ is hydrogen or methyl, are haptenic oligosaccharides, which can be used as specific inhibitors of antigenantibody reactions to characterize antigen or antibody specificities with regard to the Chlamydia-specific epitope.

The other oligomers of the invention wherein $R^1$ is a residue comprising an allyl group can be used to prepare high-molecular weight derivatives. These derivatives can be homopolymerized or copolymerized by conventional methods (Cherniak A. Y., A. B. Levinsky, B. A. Dimitriev, and N. K, Kochetkov. 1984), for example copolymerized with acrylamide or bisacrylamide to yield linear or cross-linked polymeric compounds, respectively, which are hydrophilic in nature and multivalent in terms of the antigenic determinants present. By known methods (Lee R. T. and Y. C. Lee, 1974. Carbohydr. Res. 37 : 193-201), hydrocarbon spacers can be introduced which, in dependency from the length of the $C_{1-18}$-alkyl group, endowe the molecule with hydrophobic properties useful for many serological techniques.

The oligosaccharides of the present invention, wherein $R^1$ is a $C_{1-18}$-alkyl group, which comprises a terminal hydroxyl, amino carboxyl or carbonyl group, may be reacted with amino, hydroxyl or carboxyl functions in proteins, so that the oligosaccharides can be coupled to inert proteins, such as albumin, gamma-globulin, diphteria or tetanus toxoid or others to yield glyco-protein conjugates.

These high-molecular weight derivatives being multivalent with regard to the attach-ed oligosaccharide can be used as immunogens and antigens.

For example animals and humans can be actively vaccinated to be protected against chlamydial infections. Animals and humans can be immunized to yield high titered antisera against Chlamydiae. These antisera can be used in the diagnosis and therapy (passive vaccination) of chlamydial infections in animals and humans. Animals or humans can be immunized to yield specifically primed B-lymphocytes to produce monoclonal antibodies against Chlamydiae which may be useful in the diagnosis and therapy of chlamydial infections in animals and humans.

The hydrophilic allyl copolymers can be used in immune-precipitation, immuneelectrophoresis and complement-fixation. The hydrophobic derivatives and the glyco-protein-conjugates can be used in serological assays by absorption to erythrocytes (passive hemagglutination and passive hemolysis), absorption to nitrocellulose or similar papers (western-blot techniques), coating of plastic surfaces (ELISA- and radioimmuno-techniques) or other techniques. These serological reactions can be used in the diagnosis of chlamydial infections in humans and animals. They can be further used to define the epitope specificities of monoclonal antibodies or polyclonal antisera against Chlamydiae to be used in the diagnosis or therapy of chlamyidal infections in animals and humans. From the derivatives immunoabsorbents can be prepared by coupling them to immobilized carriers such as alkylated porous glass, polyol silica, activated sepharose, activated cellulose, activated polyacrylamide, activated agarose (all commerically available) which can be used to purify monoclonal antibodies from culture fluid supernatants or ascites or to isolate specific antibody from hyperimmune sera.

The oligosaccharides of the present invention therefore are highly useful compounds, which may easily be prepared by chemical synthesis or by isolation from chlamydial or recombinant lipopolysaccharide and which have a highly interesting utility both for research and development in the medical and pharmaceutical field.

A more complete understanding can be obtained by reference to the following example and the drawings referred to therein.

**Short description of the drawings**

**Fig. 1** Shows a schematic gas-liquid chromatrogram of the chlamydial or recombinant lipopolysaccharide hydrolysate.

**Fig. 2 and 3** Show the chemical ionisation-mass spectrum with ammonia and the electron impact-mass spectrum respectively of the disaccharide.

**Fig. 4** Shows the formula of the disaccharide of the invention wherein $R^1$ is hydrogen, i.e. 8-O-(3-deoxy-D-manno-2-octulopyranosyl)onate-3-deoxy-D-manno-2-octulosonic acid.

**Fig. 5 and 6** Show the chemical ionisation-mass spectrum with ammonia and the electron impact-mass spectrum respectively of the trisaccharide and

**Fig. 7** Shows the formula of the trisaccharide of the invention wherein $R^1$ is hydrogen, i.e. 4-O-{[8-O-(3-deoxy-D-manno-2-octulopyranosyl)onate]-(3-deoxy-α-D-manno-2-octulopyranosyl)onate}-3-deoxy-D-manno-2-octulosonic acid.

**Example**

Material and methods

Bacteria and bacterial lipopolysaccharide

Salmonella minnesota rough mutant chemotype Re (strain R595) was transformed with plasmid pFEN207 which contains a 6.5 kb insert of Sau 3A-digested DNA from chlamydia trachomatis (Nano, F. E., and H. D. Caldwell. 1985 Science 228 : 742744). Bacteria were grown in a 14 l fermenter in the presence of ampicillin (80 µg/ml). Bacteria containing the plasmid pUC8 without the chlamydial insert were grown in parallel. Lipopolysaccharide was extracted from the recombinant and the control strain by the phenol-chloroform-petroleum ether method (Galanos, C., O. Lüderitz, and O. Westphal. 1969. Eur. J. Biochem. 9 : 245-249), purified by repeated ultracentrifugation, and converted to the uniform triethylammonium salt after electrodialysis (Galanos, C., and O. Lüderitz. 1975. Eur. J. Biochem. 54 : 603-610). The extracted lipopolysaccharide from the recombinant strain will be referred to as R595-207, that of the control as R595-pUC8.

The lipopolysaccharide of Chmamydia psittaci was prepared from purified yolk sacgrown elementary bodies. Chlamydia psittaci strain PK5082 of enzootic abortion in ewes was propagated in embryonated eggs as described (Sadecky, E., M. Travnicek, J. Balascak, R. Brezina, J. Kazar, J. Urvölgyi, I. Flesar, and J. Cizmar. 1978. Vet. Med.(Praha). 23 : 25-28). Infected yolk-sacs from 600 eggs were suspended in 1 M potassium chloride (20%, v/v) and inactivated with 0.5% formalin, followed by high-speed centrifugation (20,000 x g for 40 min.) The sediment was resuspended in 500 ml of phosphate-buffered saline (PBS) digested with trypsin at 37°C for 1 h with stirring. After the addition of isotonic saline (800 ml), the suspension was extracted with an excess of ethyl ether in a separating funnel. The aqueous phase was centrifuged as before, the sediment was resuspended in distilled water, dialyzed against distilled water and lyophilized. The yield of purified elementary bodies was 640 mg. Lipopolysaccharide was extracted by the phenol chloroform-petroleum ether method as modified recently (Nurminen, M., E. Th. Rietschel, and H. Brade. 1985. Infect. Immun. 48 : 573-575). The lipopolysaccharide was purified by repeated ultracentrifugation and converted to the uniform triethylammonium salt after electrodialysis.

Preparataion of cabonyl-reduced and permethylated oligosaccharides and methylation analysis.

Lipopolysaccharide from the recombinant and the control strain (50 mg each) was hydrolyzed (70°C/1h) in acetate buffer (5 ml ; 20 mM, pH 4.4). The sample was dialyzed three times against water (20 ml each) and the dialysates were combined. To the retentate, acetate buffer (1 M) was added to a final concentration of 20 mM and hydrolysis was continued for another 2h at 70°C. The sample was dialyzed as before, and the combined dialysates (containing a mixture of mono- and oligosaccharides) were neutralized with sodium hydroxide, lyophilyzed, dissolved in a small volume of water (1 ml), carbonyl-reduced with sodium borohydride or sodium borodeuteride (50 mg) at room temperature for 1 h. The reaction was quenched by the addition of a cation-exchanger resin ($H^+$-form, Bio-Rad), the solution was passed through a pasteur pipette filled with glass wool and lyophilyzed. The dry samples were permethylated according to the method of Hakomori (Hakomori, S. 1964. J. Biochem. (Tokyo) 55 : 205-208) as modified (Tacken, A., H. Brade, F. M. Unger, and D. Charon. 1986. Carbohydr. Res. in press). Methylation analysis was performed as described in detail.

Gas-liquid chromatography (GLC) and combined gas-liquid chromatography/mass spectrometry (GLC-MS).

GLC was performed on a Varian gas chromatograph model 3700 equipped with a flame-ionization detector using $H_2$ as carrier gas. A fused silica capillary column (25 m, 0,32 mm i.d.) with a chemically bonded separating phase (SE54, 0.2 µm film thickness ; Weeke Mühlheim, FRG) was used. Temperature program : 150°C for 5 min followed by an increase of 5°C/min to a final temperature of 300°C. GLC-MS was carried out on a Hewlett Packard instrument (model 5985) equipped with an SE54 column as before. Electron impact (EI) spectra were recorded at 70 eV ; chemical ionization (CI) was achieved with ammonia as reactant gas. The ion source temperature was 200°C. Spectra were processed by a Hewlett Packard data system (HP1000).

Serological methods

Monoclonal antibody L2I-6 was the one described by Caldwell and Hitchcock (Caldwell, H. D., and P. J. Hitchcock, 1984. Infect. Immun. 44 : 306-314). It recognizes a genus-specific epitope of chlamydial lipopolysaccharide. The passive hemolysis and passive hemolysis inhibition assay were performed as reported (Brade, L., M. Nurminen, P.H. Mäkelä, and H. Brade. 1985. Infect. Immun. 48 : 569572). For sen-

sitization of sheep erythrocytes, de-O-acylated lipopolysaccharide (LPS-OH) was prepared by treatment with sodium methylate (0.5 M, 37°C/16h).

Results

A) Demonstration of the chlamydia-specific epitope on recombinant lipopolysaccharide R595-207.

1. Sheep erythrocytes coated with LPS-OH from R595-207 or Chlamydia psittaci were lyzed by monoclonal antibody L2I-6, whereas those coated with LPSOH from the control (R595-pUC8) were not lyzed.
2. Lipopolysaccharide from R595-207 and from Chlamydia psittaci inhibited the antigen-antibody system of Chlamydia psittaci LPS-OH/monoclonal antibody L2I-6 with 2 ng whereas the control (lipopolysaccharide R595-pUC8) did not inhibit this system (inhibition value of > 1000 ng).

It is therefore clear, that lipopolysaccharide R595-207 contains the chlamydia-specific epitope and behaves serologically identical to the authentic lipopolysaccharide from Chlamydia psittaci, whereas lipopolysaccharide R595-pUC8 does not contain the chlamydia-specific epitope.

B) Determination of the Polysacchariae structures in lipopolysaccharides R595-207 and R595-pUC8

The polysaccharide component of both lipopolysaccharides was analyzed by a previously reported methodology (Brade, H., H. Moll, and E. Th. Rietschel. 1985. Biomed. Mass Spectrom 12 : 602-609). The derivatives obtained after acid hydrolysis, carbonyl-reduction, and permethylation were investigated by gas liquid chromatography and gas liquid chromatograpy / mass spectometry. A schematic gas-liquid chromatogram is shown in **Fig. 1**.

1. Peaks 1 and 2 represent reduced and permethylated KDO as compared with authentic standards (Brade, H. 1985. J. Bacteriol. 161 : 795-798).
2. Peak 3 represents α 2.4-linked KDO disaccharide (carbonyl-reduced and permethylated) as compared to authentic standards (Brade, H., U. Zähringer, E. Th. Rietschel, R. Christian, G. Schulz, and F. M. unger 1984. Carbohydr. Res. 134 : 157-166 ; Brade, H. and E. Th. Rietschel, 1984. Eur. J. Biochem. 145 : 213-236).

Peaks 1, 2 and 3 were observed in samples derived from lipopolysaccharides R595-pUC8 and R595-207. Therefore, these components cannot be responsible for the expression of the chlamydia-specific epitope.

3. Peak 4 was identified as dimethyl 8- O-(3-deoxy-4,5,7,8-tetra-O-methyl-D-manno-2-octulopyranosyl)onate-3-deoxy-2,4,5,6,7-penta-O-methyl-D-glycero-D-talo/galacta-octonate-2-[$^2$H].

This was shown by

i) Chemical ionisation – mass spectrometry with ammonia yielding a pseudomolecular ion peak at m/z = 633 [M + 18(ammonia)] indicating a molecular weight of M = 615 **(Fig. 2)**,
ii) Electron impact – mass spectrometry yielding the characteristic fragmentation pattern shown in **(Fig. 3)**, and
iii) Methylation analysis yielding 2,6-di-O-acetyl-3-deoxy-1,4,5,7,8-penta-O-methyl-D-glycero-D-talo/glacto-octitol- and 8-O-acetyl-3-deoxy- 1,2,4,5,6,7-hexa-O-methyl-D-glycero-D-talo/galacto-octitol-2-[$^2$H].

Therefore, peak 4 represents the derivatization product of the disaccharide 8-O-(3-deoxy-D-manno-2-octulopyranosyl)onate-3-deoxy-D-manno-2-octulosonic acid **(Fig. 4)** after carbonyl-reduction and permethylation.

4. Peak 5 was identified as trimethyl 4-O-{8-O-[(3-deoxy-4,5,7,8-tetra-O-methylD-manno-2-octulopyranosyl)onate]-(3-deoxy-4,5,7-tri-O-methyl-α-D-manno-2-octulopyranosyl)onate}-3-deoxy-2 ,5,6,7,8-penta-O -methyl-D-glycero-D-talo/galacto-octonate-2-[$^2$H].

This was shown by

i) Chemical ionisation – mass spectometry with ammonia yielding a pseudomolecular ion peak at m/z = 909 [M + 18(ammonia)] indicating a molecular weight of M = 891 **(Fig. 5)**,
ii) Electron impact – mass spectrometry yielding the characteristic fragmentation pattern shown in **(Fig. 6)**, and
iii) Methylation analysis yielding 2,4,6-tri-O-acetyl-3-deoxy-1,5,7,8-tetra-O-methyl-D glycero-D-talo/ga-

lacto-octitol, 2,6-di- O-acetyl-3-deoxy-1,4,5,7,8-penta-O-methyl-D-glycero-D-talo/galacto-octitol, and 8-O-acetyl-3-deoxy-1,2,4,5,6,7-hexa-O-methyl-D-glycero-D-talo/galacto-octitol-2-[$^2$H].

Therefore, peak 5 represents the derivatization product of the trisaccharide 4-O-{[8-O-(3-deoxy-D-manno-2-octulopyranosyl)onate]-(3-deoxy-α-D-manno-2-octulopyranosyl)onate}-3-deoxy-D-manno-2-octulo sonic acid (**Fig. 7**). After carbonyl-reduction and permethylation Peaks 1-5 were also observed in similarly prepared samples derived from authentic lipopolysaccharide of chlamydia psittaci.

Conclusion

1. The oligosaccharides of the invention shown in **Fig. 4 and 7** are compounds being only detected in the lipopolysaccharide of R595-207 and Chlamydia.These oligosaccharides are not present in the lipopolysaccharide of R595pUC8. Thus, the chlamydia-specific epitope recognized by monoclonal antibody L2I-6 is embedded in these structures.
2. The lipopolysaccharides from Chlamydia psittaci and the recombinant strain R595-207 behave chemically and serologically similar with regard to the KDO region and can substitute each other.

## Claims

### Claims for the Contracting States : BE CH LI DE FR GB SE

1. Oligosaccharides having the general formula (I)

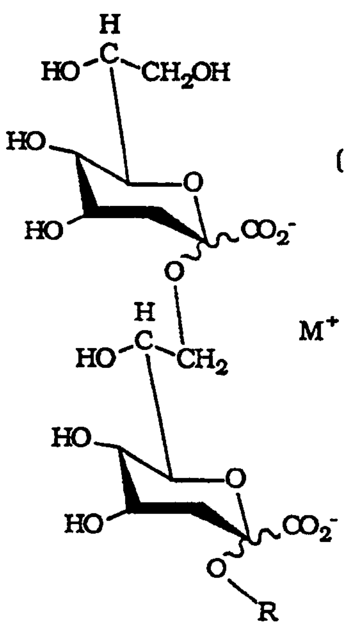

(I)

wherein R is R$^1$ or a group having the formula (II)

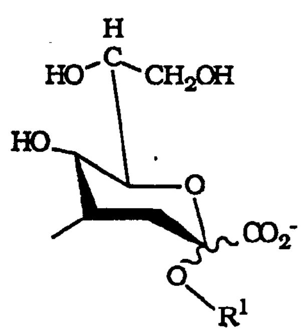

(II)

wherein R$^1$ is hydrogen or straight or branched $C_{1-18}$-alkyl, which may comprise a terminal hydroxyl, amino, carbonyl, carboxyl or allyl group, and M$^+$ is a neutralizing cation, the glycoprotein conjugates thereof

obtained with albumin, gamma-globulin or diphteria or tetanus toxoids and the polymer and copolymer derivatives of the compounds wherein $R^1$ comprises an allyl group.

2. The oligosaccharides according to claim 1, **characterised in** that $R^1$ is hydrogen, methyl or allyl.

3. The olalgosaccharides according to claim 1, **characterised in** that the neutralizing cation is a sodium, potassium, calcium or an ammonium cation.

4. A method for the manufacture of the ollgosaccharides according to claims 1 to 3, **characterized by** hydrolyzing a uniform salt of chlamydial lipopolysaccharide in an acetate buffer, dialyzing the hydrolysate against water, neutralising the dialysate, separating the oligosaccharides by liquid chromatography (gel-permeation, ion-exchange) or high-voltage paper electrophoresis (following the purity by gas-liquid chromatography and mass spectrometry) and if desired introducing the substituent $R^1$ into the disaccharide and trisaccharide obtained respectively by a method known per se and/or polymerizing or copolymenzing the derivatives of the compounds wherein $R^1$ comprises an allyl group.

5. The method according to claim 4, **characterized in** that the uniform salt of lipopolysaccharide is obtained by growing Chlamydia trachomatis or Chlamydia psittaci in tissue cultures or embriaryonated eggs or by growing recombinant bacteria which express in their lipopolysaccharide the genus-specific epitope of chlamydial lipopolysaccharide in a fermenter, extracting the lipopolysaccharide from the purified elementary bodies or recombinant bacteria respectively by the phenol-chloroform-petroleum ether method, purifying the extracted lipopolysaccharide by repeated ultracentrifugation and converting the purified lipopolysaccharide to the uniform triethylammonium salt after electrodialysis.

6. The use of oligosaccharides according to claims 1 to 3 for the manufacture of reagents and compositions for the diagnosis and therapy of human and animal chlamydial infections.

**Claims for the Contracting State : AT**

1. A method for the manufacture of oligosaccharides having the general formula (I)

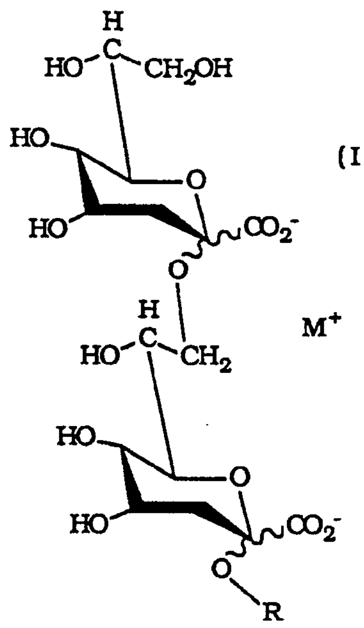

(I)

wherein R is $R^1$ or a group having the fonmula (II)

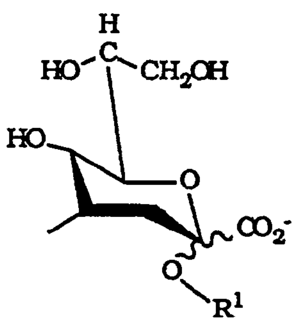

(II)

wherein $R^1$ is hydrogen or straight or branched $C_{1-18}$-alkyl, which may comprise a terminal hydroxyl, amino, carbonyl, carboxy or allyl group, and $M^+$ is a neutralizing cation, the glycoprotein conjugates thereof obtained with albumin, gamma- -globulin or diphteria or tetanus toxoids and the polymer and copolymer derivatives of the compounds wherein $R^1$ comprises an allyl group, **characterized by** hydrolyzing a uniform salt of chlamydial lipopolysaccharide in an acetate buffer, dialyzing the hydrolysate against water, neutralising the dialysate, separating the oligosacchandes by liquid chromatography (gel-permeation, ion-exchange) or high-voltage paper electrophoresis (following the purity by gas-liquid chromatography and mass spectrometry) and if desired introducing the substituents $R^1$ into the disacchari and sitraccharide obtained respectively by a method known per se and/or polymerizing or copolymerizing the derivatives of the compounds wherein $R^1$ comprises an allyl group.

2. The method according to claim 1, **characterized in** that the uniform salt of lipopolysaccharide is obtained by growing Chlamydia trachomatis or chlamydia psittaci in tissue cultures or embryonated eggs or by growing recombinant bacteria which express in their lipopolysaccharide the genus-specific epitope of chlamydial lipopolysaccharide in a fermenter, extracting the lipopolysaccharide from the purified elementary bodies or recombinant bacteria respectively by the phenol-chloroform-petroleum ether method, purifying the extracted lipopolysaccharide by repeated ultracentrifugation and converting the purified lipopolysaccharide to the uniform triethylammonium salt after electrodialysis.

3. The method accoding to claims 1 and 2, **characterized in** that oligosaccharides are prepared wherein $R^1$ is hydrogen, methyl, or allyl.

4. The method according to claims 1 and 2, **characterized in** that the neutralizing cation is a sodium potassium, calcium or an ammonium cation.

5. The use of the oligosaccharides obtained according to the method of claims 1 to 4 for the manufacture of reagents and compositions for the diagnosis of human and animal chlamydial infections.

## Revendications

### Revendications pour les Etats Contractants BE, CH, LI, DE, FR, GB, SE, LU, NL, IT

1. Oligosaccharides de formule générale (I) :

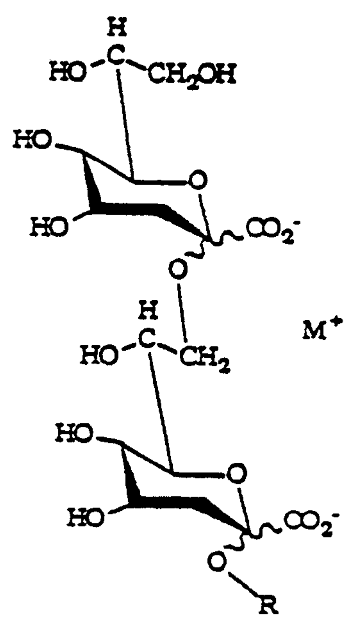

(I)

dans laquelle R représente un groupe $R^1$, ou un groupe de formule (II)

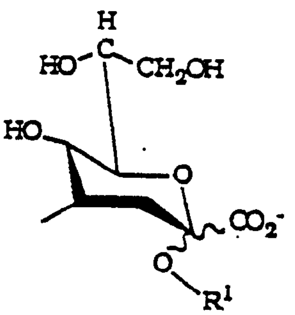

dans laquelle $R^1$ représente un atome d'hydrogène ou un groupe alkyle linéaire ou ramifié en $C_1$ à $C_{18}$ qui peut comprendre un groupe terminal hydroxyle, amino, carboxyle, carbonyle ou allyle, et $M^+$ représente un cation neutralisant ; leurs conjugués glycoprotéiques obtenus avec l'albumine, une gamma-globuline, ou l'anatoxine de la diphtérie ou du tétanos, ainsi que les polymères et les copolymères dérivés des composés dans lesquels $R^1$ comprend un groupe allyle.

2. Oligosaccharides selon la revendication 1, caractérisés en ce que $R^1$ représente un atome d'hydrogène, un groupe méthyle ou allyle.

3. Oligosaccharides selon la revendication 1, caractérisés en ce que le cation neutralisant, est un cation sodium, potassium, calcium ou ammonium.

4. Procédé de fabrication des oligosaccharides selon les revendications 1 à 3, caractérisé en ce qu'on hydrolyse un sel uniforme de lipopolysaccharide de chlamydia dans un tampon à base d'acétate, on dialyse l'hydrolysat contre de l'eau, on neutralise le dialysat, on sépare les oligosaccharides par chromatographie en phase liquide (perméation de gel, échange d'ions) ou électrophorèse à haute tension sur papier (en fonction de la pureté déterminée par chronatographie gaz-liquide et spectrométrie de masse) et, si on le souhaite, on introduit selon un procédé connu en soi, le substituant $R^1$ respectivement dans le disaccharide et le trisaccharide, et/ou on polymérise ou on copolymérise les dérivés des composés dans lesquels $R^1$ comprend un groupe allyle.

5. Procédé selon la revendication 4, caractérisé en ce que le sel uniforme de lipopolysaccharide, est obtenu en cultivant Chlamydia trachomatis ou Chlamydia psittaci dans des cultures tissulaires ou des oeufs embryonnés, ou en cultivant dans un fermenteur des bactéries recombinantes qui expriment dans leur lipopolysaccharide, l'épitope spécifique du genre du lipopolysaccharide de chlamydia, on extrait le lipopolysaccharide respectivement à partir des corps élémentaires ou des bactéries recombinantes purifiés, selon le procédé mettant en oeuvre un mélange de phénol, de chloroforme et d'éther de pétrole, on purifie le lipopolysaccharide extrait par ultracentrifugation répétée, et on convertit le lipopolysaccharide purifié en le sel uniforme de triéthylammonium après électrodialyse.

6. Utilisation des oligosaccharides selon les revendications 1 à 3, pour la fabrication de réactifs et de compositions pour le diagnostic et la thérapie de chlamydioses chez l'homme et l'animal.

**Revendications pour l'Etat Contractant : AT**

1. Procédé de fabrication d'oligosaccharides de formule générale (I) :

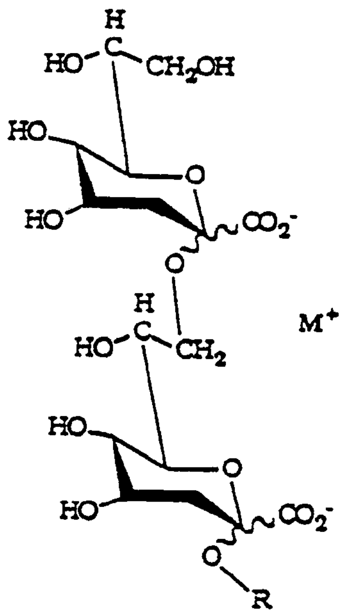

(I)

dans laquelle R représente un groupe $R^1$ ou un groupe de formule (II) :

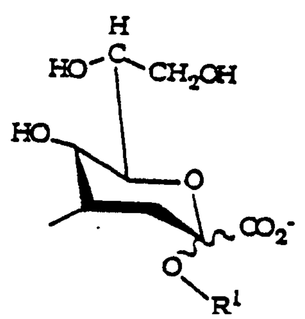

dans laquelle $R^1$ représente un atome d'hydrogène, ou un groupe alkyle linéaire ou ramifié en $C_1$ à $C_{18}$ qui peut comprendre un groupe terminal hydroxyle, amino, carboxyle, carbonyle ou allyle, et $M^+$ représente un cation neutralisant ; leurs conjugués glycoprotéiques obtenus avec l'albumine, une gamma-globuline ou une anatoxine de la diphtérie ou du tétanos, et les polymères et les copolymères dérivés des composés dans lesquels $R^1$ comprend un groupe allyle, caractérisé en ce qu'on hydrolyse un sel uniforme de lipopolysaccharide de chlamydia dans un tampon à base d'acétate, on dialyse l'hydrolysat contre de l'eau, on neutralise le dialysat, on sépare les oligosaccharides par chromatographie en phase liquide (perméation de gel, échange d'ions) ou électrophorèse à haute tension sur papier (en fonction de la pureté déterminée par chromatographie gaz-liquide et spectrométrie de masse) et, si on le souhaite, on introduit selon un procédé connu en soi, les substituants $R^1$ respectivement dans le disaccharide et le trisaccharide obtenus, et/ou on polymérise ou on copolymérise les dérivés des composés dans lesquels $R^1$ comprend un groupe allyle.

2. Procédé selon la revendication 1, carctérisé en ce le sel uniforme de lipopolysaccharide est obtenu en cultivant Chlamydia trachomatis ou Chlamydia psittaci dans des cultures tissulaires ou des oeufs embryonnés, ou en cultivant dans un fermenteur, des bactéries recombinantes qui expriment dans son lipopolysaccharide, l'épitope spécifique du genre du lipopolysaccharide de chlamydia, on extrait le lipopolysaccharide respectivement à partir des corps élémentaires et des bactéries recombinantes purifiés, selon le procédé mettant en oeuvre un mélange de phénol, de chloroforme et d'éther de pétrole, on purifie le lipopolysaccharide extrait par ultracentrifugation répétée, et après électrodialyse, on convertit le lipopolysaccharide purifié en le sel uniforme de triéthylammonium.

3. Procédé selon les revendications 1 et 2, caractérisé en ce que dans les oligosaccharides préparés, $R^1$ représente un atome d'hydrogène, un groupe méthyle ou allyle.

4. Procédé selon les revendications 1 et 2, caractérisé en ce que le cation neutralisant, est un cation sodium, potassium, calcium ou ammonium.

5. Utilisation des oligosaccharides obtenus selon le procédé des revendications 1 à 4, pour la fabrication

de réactifs et de compositions pour le diagnostic de chlamydioses chez l'homme et l'animal.

## Ansprüche

### Patentansprüche für die Vertragsstaaten : BE, CH, LI, DE, FR, GB, IT, LU, NL, SE

1. Oligosaccharide der allgemeinen Formel (I)

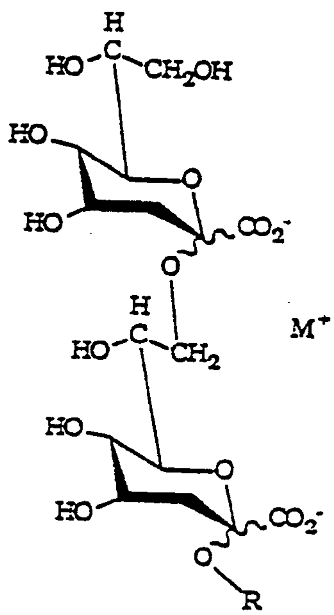

(I)

worin R $R^1$ oder eine Gruppe der Formel (II)

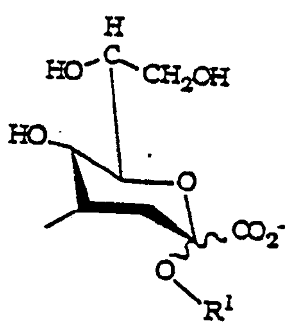

(II)

worin $R^1$ ein Wasserstoffatom oder eine geradkettige oder verzweigte $C_{1-18}$-Al-kylgruppe, die eine endständige Hydroxyl, Amino-, Carbonyl-, Carboxyl- oder Allylgruppe enthalten kann, darstellt, und $M^+$ ein neutralisierendes Kation bedeuten, deren mit Albumin, gamma-Globulin oder Diphterie- oder Tetanus-Toxoiden gebildeten Glykoproteinkonjugate und die Polymer- und Copolymerderivate der Verbindungen, worin $R^1$ eine Allylgruppe enthält.

2. Oligosaccharide nach Anspruch 1, **dadurch gekennzeichnet**, daß $R^1$ ein Wasserstoffatom, eine Methylgruppe oder eine Allylgruppe bedeutet.

3. Oligosaccharide nach Anspruch 1, **dadurch gekennzeichnet**, daß das neutralisierende kation ein Natrium-, kalium-, Calcium- oder Ammoniumkation bedeutet.

4. Verfahren zur Herstellung der Oligosaccharide nach den Ansprüchen 1 bis 3, **dadurch gekennzeichnet**, daß man ein einheitliches Chlamydienlipopolysaccharidsalz in Acetatpuffer hydrolysiert, das Hydrolysat gegen Wasser dialysiert, das Dialysat neutralisiert, die Oligosaccharide durch Flüssigkeitschromatographie (Gelchromatoraphie, Ionenaustausch) oder Hochspannungspapierelekrophorese (unter Überprüfung der Reinheit durch Gas-Flüssigkeits-Chromatographie und Massenspektrometrie) auftrennt und, falls erwünscht, den Substituenten $R^1$ nach einem an sich bekannten Verfahren in die erhaltenen Disaccharide bzw. Trisaccharide einführt und/oder die Derivate der Verbindungen, worin $R^1$ eine

Allylgruppe enthält, polymerisiert oder copolymerisiert.

5. Verfahren nach Anspruch 4, **dadurch gekennzeichnet**, daß man das einheitliche Lipopolysaccharidsalz durch Züchten von Chlamydia trachomatis oder Chlamydia psittaci in Gewebekulturen oder embryonierten Eiern oder Züchten von rekombinanten Bakterien, die in ihrem Lipopolysaccharid das genusspezifische Epitop des Chlamydienlipopolysaccharids exprimieren, in einem Fermenter, Extrahieren des Lipopolysaccharids aus den gereinigten Elementarkörpern oder den rekombinanten Bakterien nach dem Phenol-Chloroform-PetroletherVerfahren, Reinigen des extrahierten Lipopolysaccharids durch wiederholte Ultrazentnfugation und Umwandeln des gereinigten Lipopolysaccharids in das gleichformige Triethylammoniumsalz nach der Elektrodialyse erhält.

6. Verwendung der Oligosaccharide nach den Ansprüchen 1 bis 3 zur Herstellung von Reagenzien und Zusammensetzungen für die Diagnose und Therapie von Chlamydieninfekionen bei Mensch und Tier.

**Patentansprüche für den Vertragsstaat AT**

1. Verfahren zur Herstellung von Oligosacchariden der allgemeinen Formel (I)

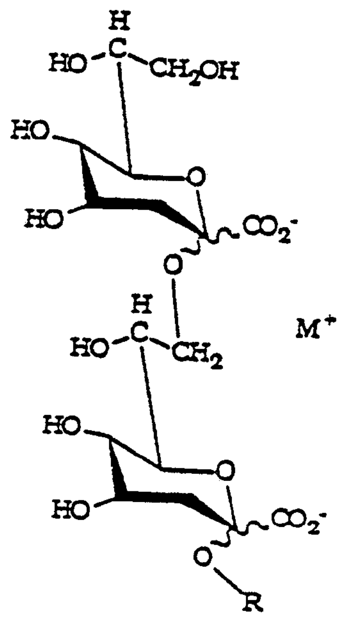

(I)

worin R $R^1$ oder eine Gruppe der Formel (II)

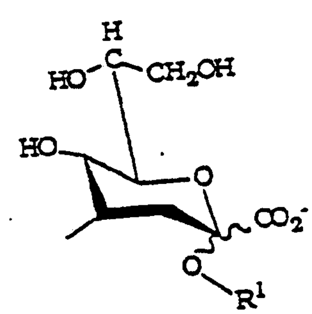

(II)

worin $R^1$ ein Wasserstoffatom oder eine geradkettige oder verzweifle $C_{1-18}$-Alkylgruppe, die eine endständige Hydroxyl-, Amino-, Carbonyl-, Carboxyl- oder Allylgruppe enthalten kann, darstellt, und M+ ein neutralisierendes Kation bedeuten, deren mit Albumin, gamma-Globulin oder Diphterie- oder Tetanus-Toxoiden gebildeten Glykoproteinkonjugaten und Polymer- und Copolymerderivaten der Verbindungen, worin $R^1$ eine Allylgruppe enthält, **dadurch gekennzeichnet**, daß man ein einheitliches Chlamydienlipopolysaccharidsalz in Acetatpuffer hydrolysiert, das Hydrolysat gegen Wasser dialysiert, das Dialysat neutralisiert, die Oligosaccharide durch Flüssigkeitschromatographie (Gelchromatographie, Ionenaustausch) oder Hochspannungspapierelektrophorese (unter Überprüfung der Reinheit durch Gas-Flüssigkeits-Chromatographie und Massenspeirtrometrie) auftrennt und, falls erwünscht, die Substituenten $R^1$ nach einem an sich bekannten Verfahren in die erhaltenen Disaccharide bzw. Trisaccharide einführt und/oder die Derivate der Verbindungen, worin $R^1$ eine alkylgruppe enthält, polymerisiert oder copolymerisiert.

2. Verfahren nach Anspruch 1, **dadurch gekennzeichnet**, daß man das einheitliche Lipopolysaccharidsahz durch Züchten von Chlamydia trachomatis oder Chlamydia psittaci in Gewebekulturen oder embryonierten Eiern oder Züchten von rekombinanten Bakterien. die in ihrem Lipopohysaccharid das genusspezifische Epitop des Chlamydienlipopolysaccharids exprimieren, in einem Fermenter, Extrahieren des Lipopolysaccharids aus den gereinigten Elementarkörpern oder den rekombinanten Bakterien nach dem Phenol-Chloroform-Petrolether-Verfahren, Reinigen des extrahierten Lipopolysaccharids durch wiederholte Ultrazentrifugation und Umwandeln des gereinigten Lipopolysaccharids in das gleichförmige triethylammoniumsalz nach der Eleirtrodialyse erhält.

3. Verfahren nach den Ansprüchen 1 und 2, **dadurch gekennzeichnet**, daß man Oligosaccharide, worin $R^1$ ein Wasserstoffatom, eine Methyl- oder Allylgruppe bedeutet, herstellt.

4. Verfahren nach den Ansprüchen 1 und 2, **dadurch gekennzeichnet**, daß das neutralisierende kation ein Natrium-, kalium-, Calcium- oder Ammoniumkation bedeutet.

5. Verwendung der nach dem Verfahren der Ansprüche 1 bis 4 erhaltenen Oligosaccharide zur Herstellung von Reagenzien und Zusammensetzungen für die Diagnose von Chlamydieninfektionen bei Mensch und Tier.

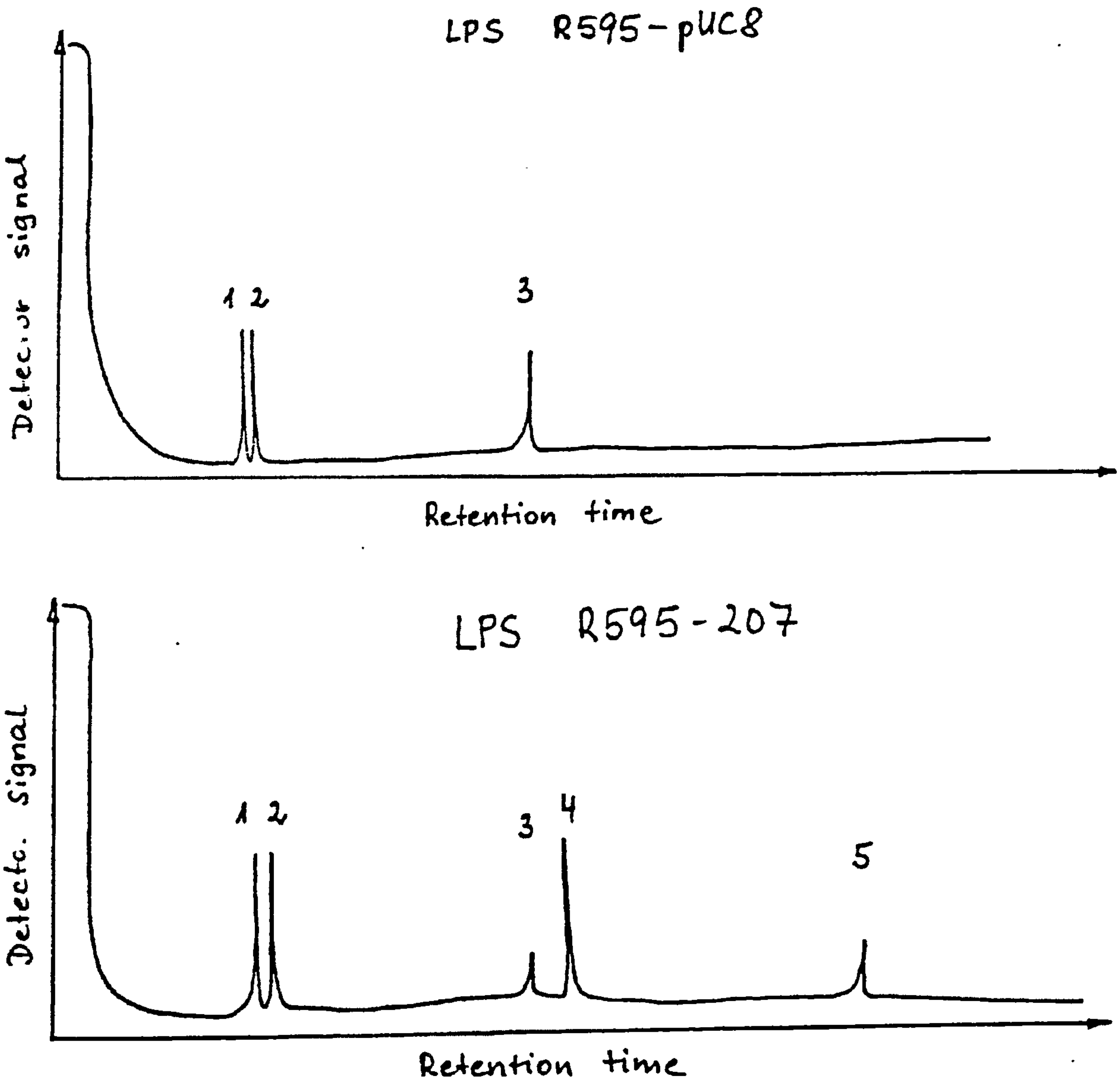
LPS R595-pUC8
Detector signal
1 2
3
Retention time
LPS R595-207
Detecto. signal
1 2
3 4
5
Retention time

Fig. 1

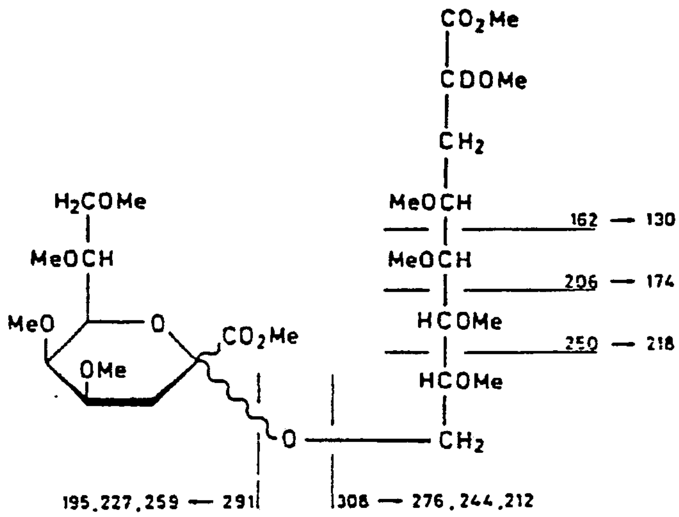
162 — 130
206 — 174
250 — 218
195,227,259 — 291
308 — 276,244,212

M = 615

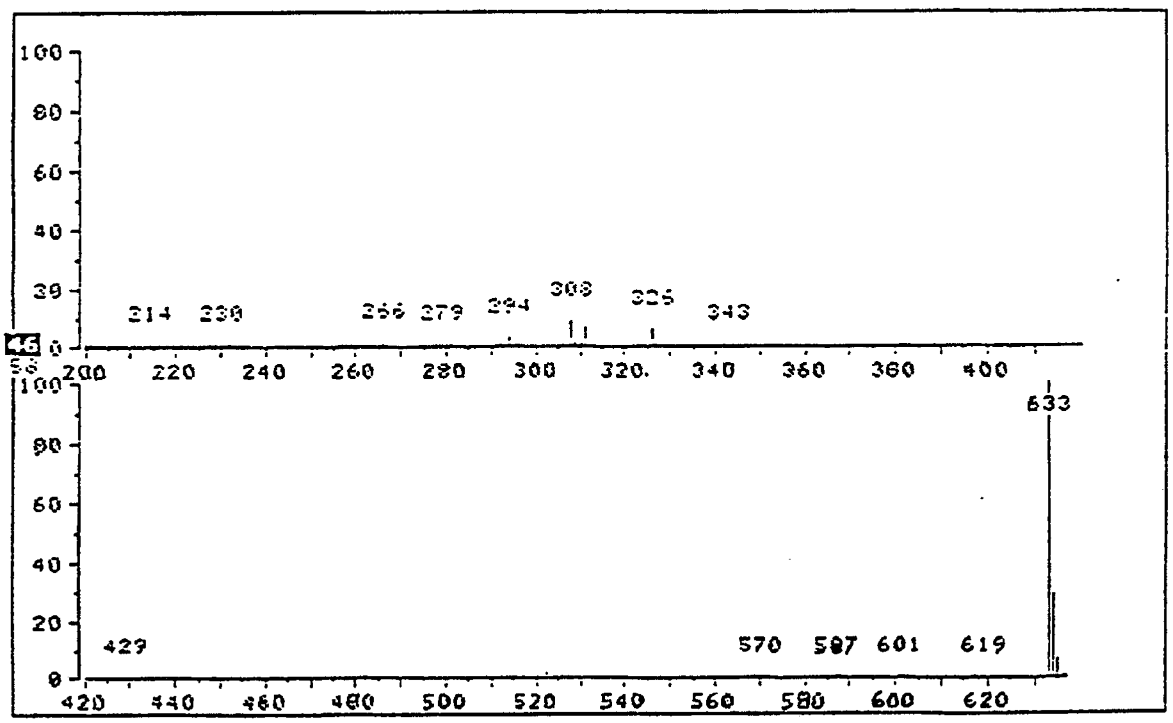
100
80
60
40
20
0
214 230 266 279 294 308 326 343
200 220 240 260 280 300 320 340 360 380 400
633
429 570 587 601 619
420 440 460 480 500 520 540 560 580 600 620

Fig. 2

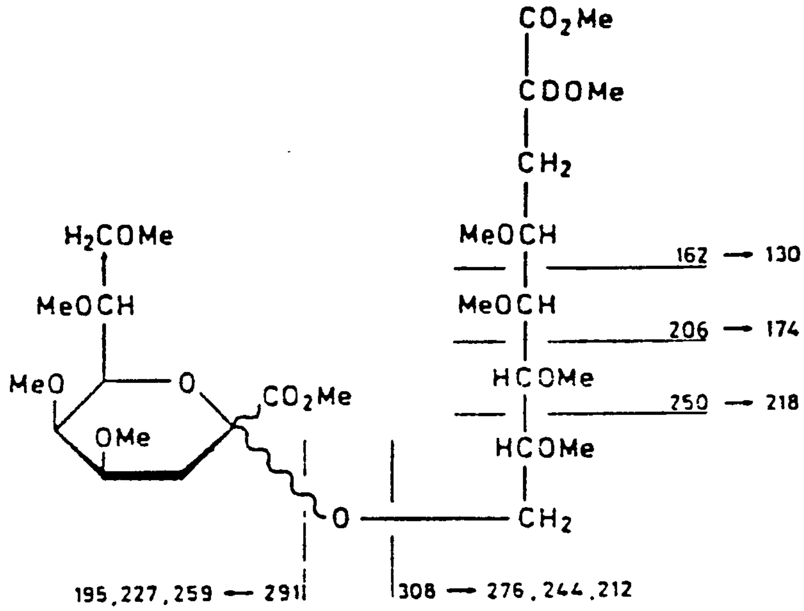
162 → 130
206 → 174
250 → 218
195, 227, 259 ← 291
308 → 276, 244, 212

M = 615

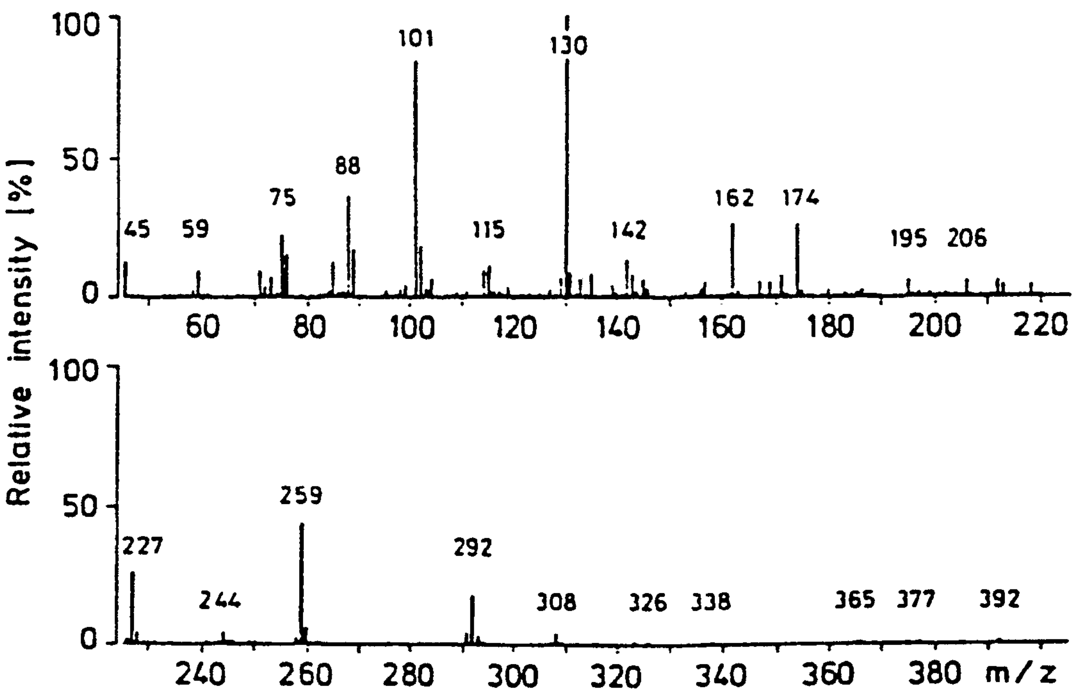
Relative intensity [%]
100
50
0
45
59
75
88
101
115
130
142
162
174
195
206
60
80
100
120
140
160
180
200
220
227
244
259
292
308
326
338
365
377
392
240
260
280
300
320
340
360
380
m/z

Fig. 3

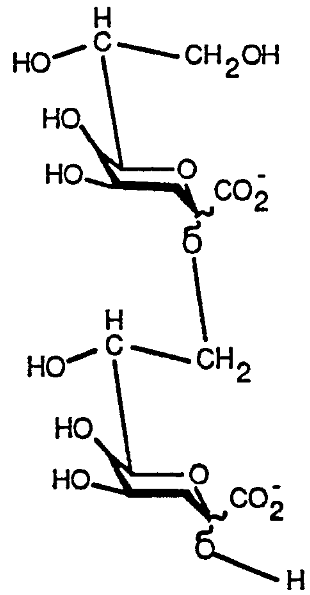

Fig. 4

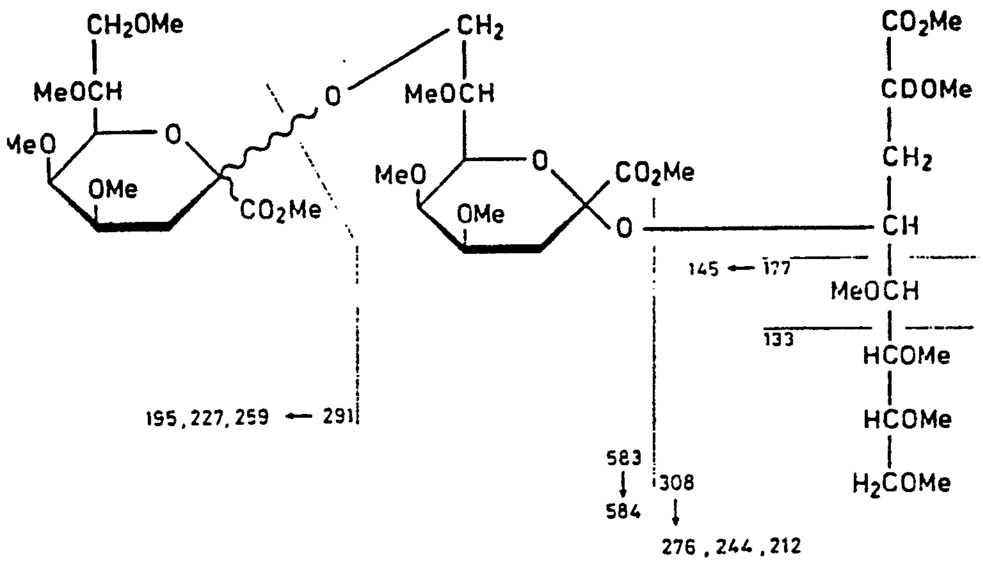
195, 227, 259 ← 291
145 ← 177
133
583 → 584
308 → 276, 244, 212

M = 891

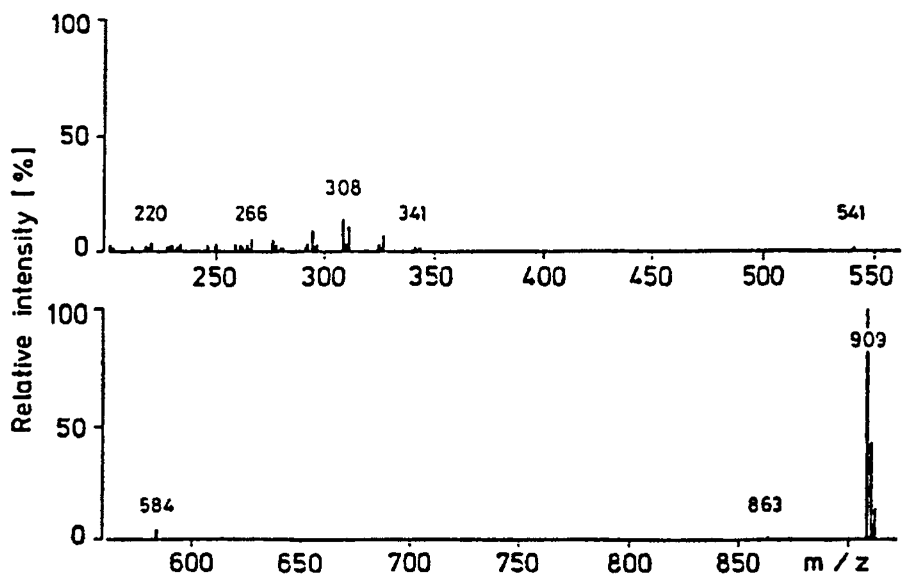
Relative intensity [%]
100
50
0
220
266
308
341
541
250
300
350
400
450
500
550
100
50
0
584
863
909
600
650
700
750
800
850
m / z

**Fig. 5**

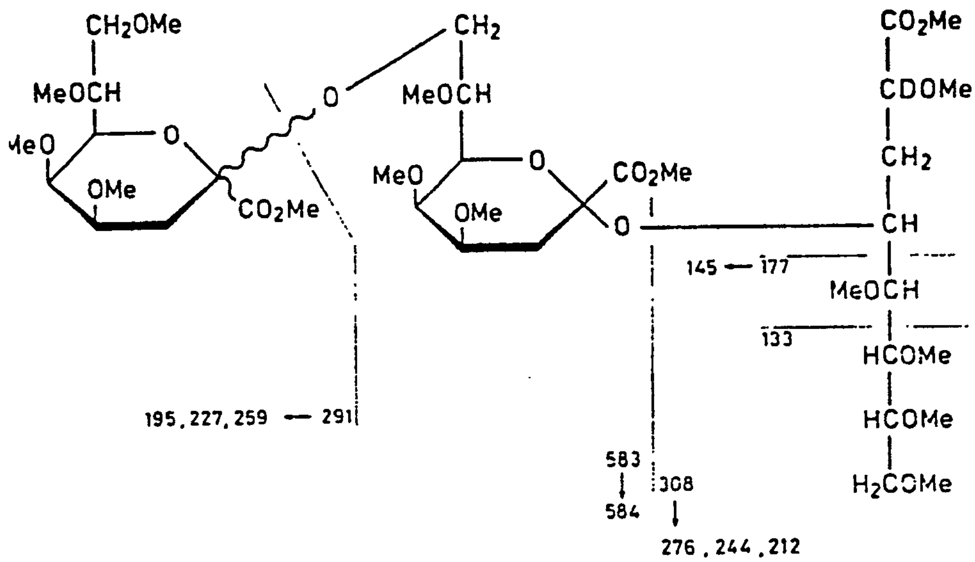
195, 227, 259 ← 291
145 ← 177
133
583
584
308
276, 244, 212

M = 891

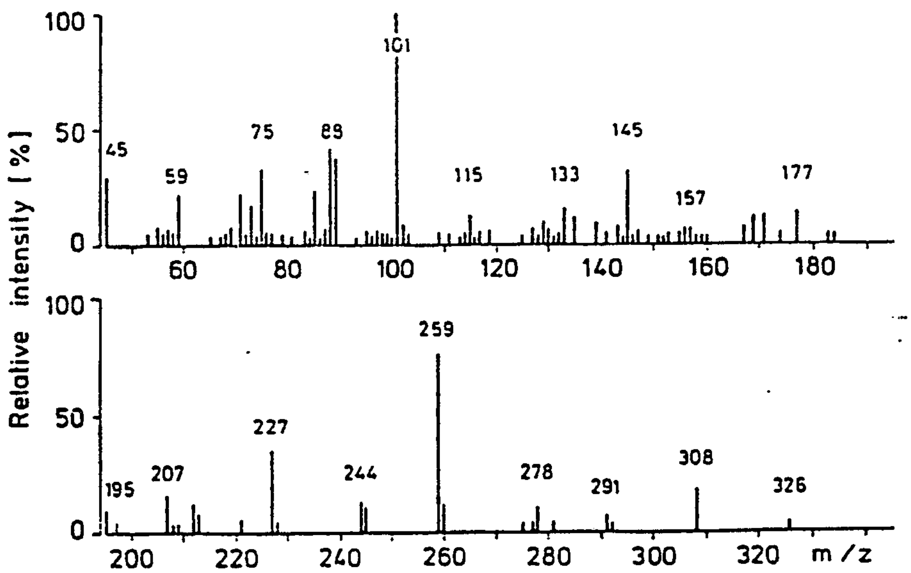
Relative intensity [%]
100
50
0
45
59
75
89
101
115
133
145
157
177
60
80
100
120
140
160
180
195
207
227
244
259
278
291
308
326
200
220
240
260
280
300
320
m/z

Fig. 6

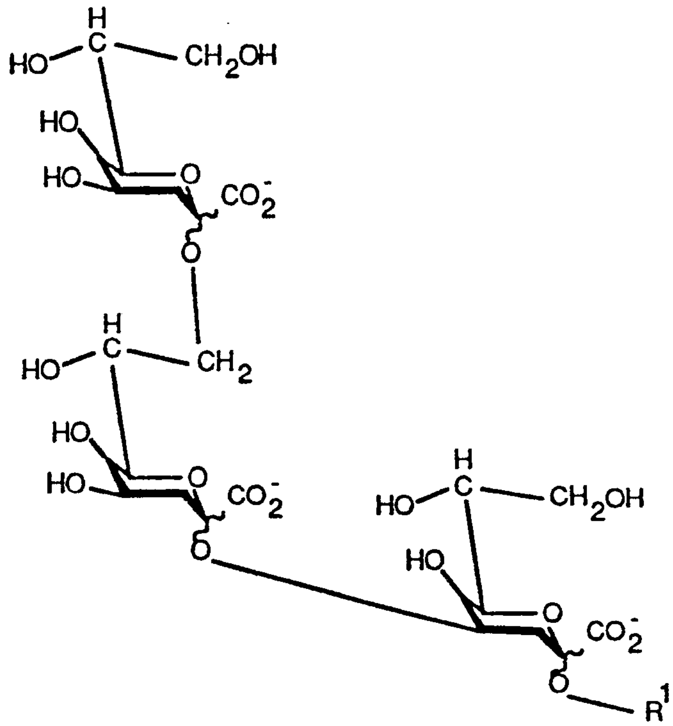

Fig. 7